(19) Europäisches Patentamt
European Patent Office
Office européen des brevets

(11) EP 4 192 420 B1

(12) EUROPEAN PATENT SPECIFICATION

(45) Date of publication and mention of the grant of the patent:
**22.05.2024 Bulletin 2024/21**

(21) Application number: **21759270.8**

(22) Date of filing: **05.08.2021**

(51) International Patent Classification (IPC):
***A61K 8/19*** (2006.01) ***A61K 8/27*** (2006.01)
***A61K 8/34*** (2006.01) ***A61K 8/41*** (2006.01)
***A61K 8/42*** (2006.01) ***A61Q 15/00*** (2006.01)

(52) Cooperative Patent Classification (CPC):
**A61Q 15/00; A61K 8/19; A61K 8/27; A61K 8/34; A61K 8/345; A61K 8/411; A61K 8/42;**
A61K 2800/31

(86) International application number:
**PCT/EP2021/071962**

(87) International publication number:
**WO 2022/029278 (10.02.2022 Gazette 2022/06)**

(54) **AN ANTIPERSPIRANT COMPOSITION**

SCHWEISSHEMMENDE ZUSAMMENSETZUNG

COMPOSITION ANTITRANSPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2020 EP 20189795**

(43) Date of publication of application:
**14.06.2023 Bulletin 2023/24**

(73) Proprietors:
- **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO SE SI SK SM**
- **Unilever Global IP Limited**
**Wirral, Merseyside CH62 4ZD (GB)**
Designated Contracting States:
**CY DE GB IE IT MT RS TR**

(72) Inventors:
- **DAS, Somnath**
**Bangalore, Karnataka 560066 (IN)**
- **SAMADDER, Satyajit**
**Bangalore, Karnataka 560066 (IN)**

(74) Representative: **Whaley, Christopher**
**Unilever Patent Group**
**Bronland 14**
**6708 WH Wageningen (NL)**

(56) References cited:
**WO-A1-2019/206764 WO-A1-2020/030414**
**DE-A1- 19 962 881**

- **B B Michniak: "Studies on the mechanism of topical anhidrosis due to polyvalent cations", InternationalJournal of Cosmetic Science, 1 January 1981 (1981-01-01), pages 29-36, XP055765254, Retrieved from the Internet: URL:n [retrieved on 2021-01-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 192 420 B1

## Description

### Field of the invention

The present invention is in the field of compositions comprising antiperspirant actives, particularly those containing zinc based actives. The present invention more particularly relates to such compositions which not only exhibit efficacy similar to or better than that of well known aluminium based actives but is delivered with a desired gel like consistency in an anhydrous carrier. The compositions of the invention are as defined in the appended set of claims.

### Background of the invention

The present invention relates to compositions, such as those that contain antiperspirant actives. These actives are added to compositions to reduce perspiration on application to the surface of the body, particularly to the underarm regions of the human body viz. the axilla. Antiperspirant actives are typically astringent metal salts such as those of aluminium or zirconium salts. Antiperspirant actives are usually incorporated in compositions at low pH, in the range of 2 to 7. There has been a thrust to develop anti perspirant actives which are less astringent and the approach has been to look for actives that do not contain aluminium. The present inventors with their extensive research in the field of zinc compounds have developed a zinc compound which is a complex of a zinc salt with specifically β amino alcohol which is found to have similar or better efficacy as compared to that of well-established aluminium based actives. Compositions containing this active in specific anhydrous carrier has been published as WO2020/030411.

WO 2020/030414 A1 (Unilever) discloses an antiperspirant composition having a complex of a zinc salt with a β amino acid in an anhydrous carrier.

WO 2019/206764 A1 (Unilever) discloses an antiperspirant composition having a cholic acid derivative and a multivalent metal salt in a topically acceptable carrier.

The paper "Studies on the mechanism of topical anhidrosis due to polyvalent cations" (B B Michniak: International-Journal of Cosmetic Science, 1 January 1981. pg. 31-33) discloses that topical application of a calcium chloride solution promotes sweating and it reduces effectiveness aluminium, lanthanum and zirconium solution when included with these compounds.

DE 19962881 A1 (Henkel) discloses an aqueous creamy antiperspirant compositions having a combination of at least two particulate polysaccharides in an oil/wax matrix which imparts to the skin a distinctive dry and smooth sensation and the composition does not leave any residues on the skin.

The present inventors wishing to deliver this in an anhydrous composition with a specific gel like consistency without any loss of antiperspirancy efficacy hit upon specific multivalent metal salts in an alcohol containing composition to achieve this.

It is thus an object of the present invention to provide for an antiperspirant composition which exhibits high antiperspirant efficacy while ensuring that it is delivered through a gel like composition meeting certain viscosity properties.

### Summary of the invention

According to the first aspect of the present invention there is provided an antiperspirant composition comprising

(i) a complex of a zinc salt with a β amino alcohol;
(ii) an anhydrous solvent comprising an alcohol; and
(iii) a water soluble multivalent metal salt wherein the multivalent metal salt is calcium chloride.

According to another aspect of the present invention there is provided a non therapeutic method of reducing perspiration comprising the step of applying a composition as claimed in the first aspect on to the desired skin surface.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical

properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The compositions of the invention are typically "personal care compositions", suitable for cosmetic use as detailed below. Further, use of the compositions of the invention is typically cosmetic, non-therapeutic use.

The compositions may be used for the therapeutic treatment of hyperhidrosis (extreme sweating).

By "An Antiperspirant Composition" as used herein, is meant to include a composition for topical application to the skin of mammals, especially humans. Such a composition is preferably of the leave-on type. By a leave-on composition is meant a composition that is applied to the desired skin surface and left on for a period of time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be an anhydrous composition which could be in the form of a gel or in stick form or may be delivered through a roll-on device. It is especially useful for delivering compositions to the axilla of an individual for anti-perspirancy benefits. "Skin" as used herein is meant to include skin on any part of the body where one may sweat (e.g., neck, chest, back, arms, underarms, hands, legs, buttocks and scalp) especially the underarms.

The present invention is directed to delivering a zinc based antiperspirant active on to the topical surface of a human body. The active for inclusion in the composition is a complex of a zinc salt with a β amino alcohol.

Suitable salt for use in preparing the complex are a chloride, formate, acetate, propionate, gluconate or citrate of zinc.

The preferred β amino alcohol for use in preparing the complex with the zinc salt is chosen from mono ethanol amine, diethanol amine, triethanol amine or 2-amino-1-butanol.

β amino alcohols have the structure as given below:

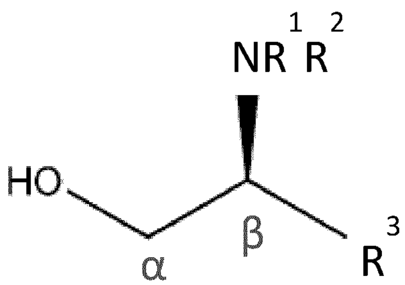

Where R1, R2 and R3 are an alkyl or aryl group which may be functionalized or is H.

When R1, R2 and R3 are functionalized, it is most preferably β-hydroxy or o-phenolic functionalized.

The structure of the preferred β amino alcohols are:

Monoethanol amine (MEA)

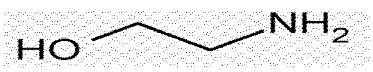

Diethanol amine (DEA)

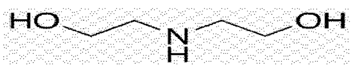

Triethanol amine

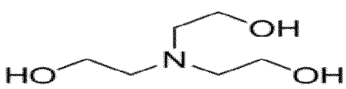

2-Amino-1-Butanol

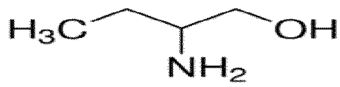

The present inventors have found that complexes of zinc salts only with β amino alcohols provide this benefit especially when incorporated in the non-aqueous carrier of the present invention. They found that this benefit is not found when α amino alcohol or γ amino alcohol are used.

The complex is so included that the amount of zinc is preferably from 0.2 to 2%, more preferably from 0.5 to 1.5% by weight of the composition.

The zinc compound and the β amino acid are preferably complexed in a molar ratio in the range of 1:0.5 to 1:3, preferably in the range of 1:1 to 1:3. The present inventors have found that the desired precipitation of the complex in aqueous media of sweat is good when the molar ratio is in the claimed range, thereby providing the desired AP activity.

The complex of zinc salt with β amino alcohol as per this invention is included in a composition comprising an anhydrous solvent which includes an alcohol. Preferred alcohols include C2 to C4 alcohol, alkylene glycol or mixtures thereof. Of the C2 to C4 alcohol, ethanol is preferred. Alkylene glycol when included in the composition of the invention is preferably a C2 to C4 alkylene glycol, preferably propylene glycol.

Optimum antiperspirant activity of the above active is found when it is delivered through an anhydrous solvent which comprises a mixture of ethanol and an alkylene glycol wherein the alkylene group comprises C2 to C4 carbon atoms. An especially suitable anhydrous solvent is a mixture of ethanol and propylene glycol. An optional solvent for inclusion in the composition is a silicone oil.

Ethanol is preferably included in 40 to 85% preferably 45 to 75% by weight of the anhydrous solvent.

The anhydrous solvent is preferably included in 20 to 90, preferably 50 to 80% by weight of the composition.

In addition to the ingredients disclosed above, the composition achieves the desired gel consistency by inclusion of a water soluble multivalent metal salt, while ensuring that the antiperspirancy efficacy of the composition is not compromised. By water soluble salt of multivalent metal is meant that the solubility of the salt in water is higher than 60 g/l at 25 °C. The multivalent metal salt is also preferably soluble in ethanol. When included in the composition of the invention, the salt is preferably anhydrous. The salt used in the present invention is calcium chloride. Calcium chloride is included in 0.2 to 6%, preferably 0.8 to 4.2% by weight of the composition. Preferably the composition includes calcium ion at 0.1 to 1.8% preferably 0.3 to 1.5% by weight of the composition.

Without wishing to be bound by theory the inventors believe that the inclusion of the multivalent metal salt increases the packing of zinc-alcoholamine complex and thus leads to a gel like structure within a fixed ratio of zinc to added multivalent metal ions

The pH of the 1% w/w aqueous dispersion of the composition is preferably in the range of 5 to 8. The present inventors have found that when the pH of the composition is lowered below 5 or increased above 8, the zinc complex of the invention is likely to be more solubilized in the aqueous medium of the sweat. Under such conditions, the antiperspirant activity is likely to be low. Therefore a pH in the range of 5 to 8 is preferred.

The complex is preferably prepared by a process comprising the steps of (i) dissolving the β amino alcohol in the anhydrous solvent; and (ii) dissolving therein the zinc salt. It is preferred that the dissolution is carried out near room temperature i.e. from about 20 °C to about 40 °C. The dissolution is preferably carried out in each of the individual steps for about 5 minutes to 30 minutes. Further preferably the solution of the complex is mixed to ensure complete dissolution from 1 hour to about 8 hours.

The complex so prepared is preferably dissolved or suspended in the anhydrous carrier during the preparation of the composition of the invention.

Antiperspirant compositions of the present invention may advantageously comprise an additional, non-zinc based antiperspirant active. Whilst this might be a conventional antiperspirant salt comprising Al and/or Zr, such as aluminium chlorohydrate or aluminium-zirconium chlorohydrate optionally complexed with glycine, it is preferred that any additional antiperspirant active is not of this type.

Other components commonly included in conventional antiperspirant compositions may also be incorporated in the compositions of the present invention. Such components include skin care agents such emollients, humectants and skin barrier promoters; skin appearance modifiers such as skin lightening agents and skin smoothing agents; antimicrobial agents, in particular organic anti-microbial agents, and preservatives.

The anti-perspirant active can be applied cosmetically and topically to the skin. In the method of the invention, the composition is wiped across the surface of the skin, depositing a fraction of the composition as it passes. The composition of the invention is preferably delivered through a skick or a gel form or using a roll on.

Stick or soft solid compositions

Many different materials have been proposed as an additional gellant for a continuous oil phase, including

waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax.

The composition of the present invention can comprise a wide range of other optional components. The CTFA Personal care Ingredient Handbook, Second Edition, 1992, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

The present invention also provides for a non-therapeutic method of reducing perspiration comprising the step of applying the composition of the first aspect on to the desired skin surface. The skin surface could be any topical surface which is prone to sweating especially the axilla i.e. the underarm portion of the human body. The method is non-therapeutic.

The invention will now be demonstrated with the help of the following examples.

**Examples**

Examples A-B, 1-4: Percentage flow rate reduction across a membrane coated with antiperspirant active and the dynamic viscosity of the compositions.

Example A: Aluminium chlorohydrate (ACH) was formulated at 12 wt% as shown in Table -1 below:

Table -1

| Ingredient | % w/w |
|---|---|
| ACH (Aloxicoll PF40) | 12.00 |
| Brij 72 | 2.60 |
| BrijS20 | 0.12 |
| Sunflower seed oil (Aldrich) | 0.40 |
| Water | 84.88 |

Example B, 1-4: Zinc alcohol amine complex was formulated as in shown in Table - 2 below for Example - 2:

Table -2

| Ingredients | % w/w |
|---|---|
| Crodamol ICS | 4.00 |
| Cetyl alcohol | 2.00 |
| Zinc acetate dihydrate | 3.35 |
| 2-Amino-1-butanol (2A1B) | 1.36 |
| SP Brij S2 MBAL | 1.73 |
| Brij S20 | 0.40 |
| Calcium chloride(anhydrous)* | 2.78 |
| Glycerol | 0.5 |
| Anhydrous solvent** | To 100 |

*Anhydrous calcium chloride at 2.78 wt% is equivalent to 1 wt% calcium ion concentration in the composition.
** Anhydrous solvent refers to a mixture of ethanol, propylene glycol and silicone oil (DC245) in a weight ratio of 6:1:1.

The compositions of the other examples B, 1, 3, 4 were formulated as in Table - 2 above except that the calcium ion concentration was varied as shown in Table - 3 hereinbelow.

In the above Table zinc alcohol amine was prepared as follows:

Step -1: Preparation of zinc acetate alcoholamine complex in the anhydrious solvent containing multivalent metal ion:

Solution-A
Required amount of 2-Amino-1-butanol, was added in absolute ethanol and was stirred for 10 mins at 600 rpm on a magnetic stirrer, to get it completely dissolved. In this solution, required amount of zinc acetate dihydrate, free-flowing powder, was added and stirred well at about 600 rpm to get a clear solution till zinc acetate dissolves completely. Required amount of glycerol was added and stirred well in the same solution. Now this ethanolic calcium chloride solution was added in a dropwise fashion (flow rate 10 ml/min) under continuous stirring.
After completion of ethanolic calcium chloride solution addition, a stable gel phase was obtained. In this required amount of DC245 was added and mixed well.

Step -2: Preparation of propylene glycol part: Solution-B
Dried propylene glycol was taken and in it required amount of Cetyl alcohol (a flaky powder), SP Brij S2 MBAL (a sticky solid), Brij S 20 (a sticky, waxy solid), and Crodamol ICS (a transparent, odor-less liquid), respectively, was added and was kept in an air oven, maintained at 50° C. A clear solution was obtained in this method.

Step -3: Preparation of final solution
In this final step, both the solutions (Solution A and Solution B) as obtained by earlier steps was added together. In the solution obtained in the step-2, i.e. Solution-B, the gel phase as obtained at step-1, i.e. Solution-A, was added under continuous stirring at 600 rpm.

The mixing operation was continued under vigorous stirring condition for 2 hrs. to ensure complete mixing of Solution A and Solution B to get a thick, soft-solid gel. After 2 hrs. of vigorous mixing, the gel phasic material was kept in an air-oven under well-stoppered condition at 55-60° C for 3 hrs. The semi-solid formulation thus obtained, after cooling to room temperature, was used for further studies to evaluate Zn-alcoholamine based antiperspirants.

For preparing Zn-alcoholamine complex coated membrane:
Whatman™ Cellulose Nitrate Membrane Filters being used having 50 mm diameter and 12-micron nominal pore size (Whatman™ 10400014 Grade AE100) was coated with the requisite volume of aqueous AP active by dropwise addition of the solution with the help of a micropipette ensuring proper spreading. The coated membranes were then placed inside high humidity (RH > 90%) for 4 hours. The membranes were taken out and kept at ambient condition (25 °C, 70%RH, loosely covered with a lid without any contact with membranes and ensuring equilibrium with ambient air) overnight.

For preparing ACH coated membrane:

Whatman™ Cellulose Nitrate Membrane Filters being used having 50 mm diameter and 12-micron nominal pore size (Whatman™ 10400014 Grade AE100) was coated with the requisite volume of aqueous AP active by dropwise addition of the solution with the help of a micropipette ensuring proper spreading. The coated membranes were then placed inside ammonia vapor blanket (10 -15 % w/w aqueous ammonium hydroxide solution kept inside a closed desiccator) for 3 hours. The membranes were taken out and kept at ambient condition (25 °C, 70%RH, loosely covered with a lid without any contact with membranes and ensuring equilibrium with room air) overnight.

The percentage flow rate reduction was calculated using the equation:

$$\text{percentage flow rate reduction (PFRR): } = \frac{[\text{ control membrane flow rate(mL/min)- coated membrane flow rate (mL/min)}]}{[\text{ control membrane flow rate(mL/min)}]} \times 100$$

The data on PFRR of the samples is given in Table - 3 below:

The dynamic viscosity was measured as follows

Dynamic viscosity was measured using an Advanced Rheometer, Model AR1000 from TA Instruments. About 1 g of the sample was applied on a clean steel plate. The rotational blade was lowered onto the sample and applied torque was adjusted to ensure mechanical deformation. The amount of torque required to cause rotation across a horizontal plane in the sample was measured.

Table - 3

| Example | Active + wt% calcium ion concentration | Avg % PFRR | Std Dev | Dynamic viscosity (Pa.s) at 25 °C. |
|---|---|---|---|---|
| A | ACH | 89 | 2 | |
| B | ZAA + 0% Calcium ion | 93 | 1 | 0.003 |
| 1 | ZAA + 0.5% Calcium ion | 81 | 1 | 2.5 |
| 2 | ZAA + 1.0% Calcium ion | 90 | 1 | 3.7 |
| 3 | ZAA + 2.0% Calcium ion | 94 | 1 | 0.3 |
| 4 | ZAA + 5.0% Calcium ion | 96 | 1 | 0.9 |

In the above table, the %PFRR was an average of N (no. of membrane, repeat) = 5

The data in Table - 3 above indicates that the compositions as per the invention (Examples 1 - 4) provides for almost the same or much better antiperspirant activity as compared to the most commonly used commercial anti perspirant active (Example A). The compositions 1-4 also provide acceptable dynamic viscosity (higher than 0.1 Pa.s) with the examples 1-2 giving the best gel like consistency.

Back Sauna test:

Examples A, B and 2 were used in a Back sauna test using a procedure as follows:

The aim of this study is to determine the perspiration-reducing efficacy of the test products in comparison with control areas under dermatological control. It is the objective to determine the absolute and relative reduction of sweating

Evaluation criteria

Days 1 to 4: On each day, the test products was applied and evenly distributed on the test area as identified in the randomisation schedule (75µl (liquids) or 75µg (solids)) for each subject.

After application the subjects rested for approximately 5 minutes to allow the test products to absorb into the skin. Afterwards all test and control areas were covered with occlusive non-absorbent patches. Subjects then left the test centre and returned 2h (± 5min) later when the patches were removed by the study technician.

Day 5: The back of the subjects was not cleaned with water, as in the standard protocol, prior to entering the sauna. This was to intentionally leave the test products in-situ. All test areas, as well as untreated contralateral areas, were covered with preweighed absorbent pads on plastic sheets (attached to the back with Fixomull®). The subjects then lay in prone position in a sauna at approximately 80°C for about 15 minutes. Directly after leaving the sauna all pads were removed with tweezers and put into cups. Gravimetrical measurements of pads were performed immediately.
The data on the mean sweat reduction is shown in Table - 4.

Table -4:

| Example | N | Mean sweat reduction (%) | p-value |
|---|---|---|---|
| A (12% ACH) | 25 | 65 | <0.002 |
| B (ZAA + 0% Ca) | 22 | <1 | 0.093 |
| 2 (ZAA + 1% Ca) | 24 | 20.4 | 0.002 |

A sweat reduction of higher than 20% is considered acceptable.
The data in the table 4 above indicates that composition as per the invention, Example - 2 gives acceptable sweat reduction at just 4.5% active concentration as compared to a conventional aluminium based composition at 12% active concentration.

## Claims

1. An antiperspirant composition comprising:

   (i) a complex of a zinc salt with a β amino alcohol;
   (ii) an anhydrous solvent comprising an alcohol; and,
   (iii) a water soluble multivalent metal salt wherein the multivalent metal salt is calcium chloride at 0.2 to 6% by weight.

2. A composition as claimed in claim 1 wherein the anhydrous solvent is selected from $C_2$ to $C_4$ alcohol, alkylene glycol or mixtures thereof.

3. A composition as claimed in claims 1 and claim 2 wherein the alkylene glycol is $C_2$ to $C_4$ glycol preferably propylene glycol.

4. A composition as claimed in claim 3 comprising a mixture of ethanol and propylene glycol.

5. A composition as claimed in claim 4 wherein ethanol is included in 40 to 85% by weight of the anhydrous solvent.

6. A composition as claimed in any one of the preceding claims wherein said zinc salt is selected from a chloride, formate, acetate, propionate, gluconate or citrate.

7. A composition as claimed in any one of the preceding claims wherein said β amino alcohol is chosen from mono ethanol amine, diethanol amine, triethanol amine or 2-amino-1-butanol.

8. A composition as claimed in any one of the preceding claims wherein the molar ratio of zinc salt to β amino alcohol is from 1:0.5 to 1:3 preferably from 1:1 to 1:3.

9. A composition as claimed in any one of the preceding claims comprising 20 to 90% anhydrous solvent by weight of the composition.

10. A composition as claimed in any one of the preceding claims wherein the complex is prepared using a process comprising the steps of

    (i) dissolving the β amino alcohol in the anhydrous solvent; and
    (ii) dissolving therein the zinc salt.

**11.** A non-therapeutic method of reducing perspiration comprising the step of applying a composition as claimed in any one of the preceding claims on to the desired skin surface.

## Patentansprüche

**1.** Schweißhemmende Zusammensetzung, umfassend:

(i) einen Komplex eines Zinksalzes mit einem β-Aminoalkohol;
(ii) ein wasserfreies Lösungsmittel, umfassend einen Alkohol; und
(iii) ein wasserlösliches mehrwertiges Metallsalz, wobei das mehrwertige Metallsalz Calciumchlorid mit einem Anteil von 0,2 bis 6 Gewichts-% ist.

**2.** Zusammensetzung, wie im Anspruch 1 beansprucht, wobei das wasserfreie Lösungsmittel unter $C_2$- bis $C_4$-Alkohol, Alkylenglycol oder Mischungen davon, ausgewählt ist.

**3.** Zusammensetzung, wie im Anspruch 1 und im Anspruch 2 beansprucht, wobei das Alkylenglycol $C_2$- bis $C_4$-Glycol, vorzugsweise Propylenglycol, ist.

**4.** Zusammensetzung, wie im Anspruch 3 beansprucht, umfassend eine Mischung von Ethanol und Propylenglycol.

**5.** Zusammensetzung, wie im Anspruch 4 beansprucht, wobei Ethanol in einer Menge von 40 bis 85%, bezogen auf das Gewicht des wasserfreien Lösungsmittels, enthalten ist.

**6.** Zusammensetzung, wie in irgendeinem der vorhergehenden Ansprüche beansprucht, wobei das Zinksalz unter Chlorid, Formiat, Acetat, Propionat, Gluconat oder Citrat ausgewählt ist.

**7.** Zusammensetzung, wie in irgendeinem der vorhergehenden Ansprüche beansprucht, wobei der β-Aminoalkohol unter Monoethanolamin, Diethanolamin, Triethanolamin oder 2-Amino-1-butanol ausgewählt ist.

**8.** Zusammensetzung, wie in irgendeinem der vorhergehenden Ansprüche beansprucht, wobei das Molverhältnis von Zinksalz zu β-Aminoalkohol 1:0,5 bis 1:3, vorzugsweise 1:1 bis 1:3, beträgt.

**9.** Zusammensetzung, wie in irgendeinem der vorhergehenden Ansprüche beansprucht, umfassend 20 bis 90% wasserfreies Lösungsmittel, bezogen auf das Gewicht der Zusammensetzung.

**10.** Zusammensetzung, wie in irgendeinem der vorhergehenden Ansprüche beansprucht, wobei der Komplex unter Anwendung eines Verfahrens hergestellt wird, umfassend die Schritte

(i) Auflösen des β-Aminoalkohols in dem wasserfreien Lösungsmittel; und
(ii) darin Auflösen des Zinksalzes.

**11.** Nicht-therapeutisches Verfahren zur Reduzierung der Schweißbildung, umfassend den Schritt des Auftragens einer Zusammensetzung, wie in irgendeinem der vorhergehenden Ansprüche beansprucht, auf die gewünschte Hautoberfläche.

## Revendications

**1.** Composition anti-transpirante comprenant:

(i) un complexe d'un sel de zinc avec un β amino alcool;
(ii) un solvant anhydre comprenant un alcool; et,
(iii) un sel métallique multivalent soluble dans l'eau, dans laquelle le sel métallique multivalent est le chlorure de calcium à raison de 0,2 à 6 % en poids.

**2.** Composition selon la revendication 1, dans laquelle le solvant anhydre est choisi parmi un alcool en $C_2$ à $C_4$, un alkylène glycol ou des mélanges de ceux-ci.

**3.** Composition selon la revendication 1 et la revendication 2, dans laquelle l'alkylène glycol est un glycol en $C_2$ à $C_4$, de préférence le propylène glycol.

**4.** Composition selon la revendication 3 comprenant un mélange d'éthanol et de propylène glycol.

**5.** Composition selon la revendication 4, dans laquelle l'éthanol est inclus à raison de 40 à 85 % en poids du solvant anhydre.

**6.** Composition selon l'une quelconque des revendications précédentes, dans laquelle ledit sel de zinc est choisi parmi un chlorure, formiate, acétate, propionate, gluconate ou citrate.

**7.** Composition selon l'une quelconque des revendications précédentes, dans laquelle ledit β amino alcool est choisi parmi la monoéthanolamine, la diéthanolamine, la triéthanolamine ou le 2-amino-1-butanol.

**8.** Composition selon l'une quelconque des revendications précédentes, dans laquelle le rapport molaire du sel de zinc au β amino alcool est de 1:0,5 à 1:3, de préférence de 1:1 à 1:3.

**9.** Composition selon l'une quelconque des revendications précédentes comprenant 20 à 90 % de solvant anhydre en poids de la composition.

**10.** Composition selon l'une quelconque des revendications précédentes, dans laquelle le complexe est préparé au moyen d'un procédé comprenant les étapes de

(i) dissolution du β amino alcool dans le solvant anhydre; et
(ii) dissolution du sel de zinc dans ceci.

**11.** Procédé non thérapeutique de réduction de la transpiration comprenant l'étape d'application d'une composition selon l'une quelconque des revendications précédentes sur la surface de peau souhaitée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020030411 A
- WO 2020030414 A1
- WO 2019206764 A1
- DE 19962881 A1


### Non-patent literature cited in the description


- **B B MICHNIAK.** Studies on the mechanism of topical anhidrosis due to polyvalent cations. *International-Journal of Cosmetic Science,* 01 January 1981, 31-33
- The CTFA Personal care Ingredient Handbook. 1992